# EUROPEAN PATENT APPLICATION

(11) **EP 3 995 805 A1**
(43) Date of publication of application: **11.05.2022**
(21) Application number: 20205704.8
(22) Date of filing: 04.11.2020
(51) Int. Cl.: G01N 1/20, C12M 1/26, C12M 1/12, A61M 39/22, A61M 1/02

(54) **A SAMPLING DEVICE WITH A SAMPLE CONTAINER AND A THREE-WAY VALVE**

(71) Applicant: Keofitt A/S, 5700 Svendborg (DK)
(72) Inventor: Borup, Susanne Bakman, 5700 Svendborg (DK); Markvardsen, Rasmus, 5700 Svendborg (DK); Salomon, Henrik Lysgaard, 5700 Svendborg (DK); Borg, Marie Bay, 2700 Brønhøj (DK); Christensen, Thomas Olund, 2800 Lyngby (DK)
(74) Representative: AWA Denmark A/S

(57) **Abstract**

A sampling device (1) comprises a sample container (3) having a flexible wall inclosing an interior (33); and a three-way valve (5) provided with a first conduit connection (51) to a first coupling member (53); a second conduit connection (55) to an outlet (57); and a connection (59) to the interior (33). The three-way valve (5) is configured to open and close a connection between the first coupling member (53) and the interior (33) of the sample container (3). The first conduit connection (51) is passing through a first section (31a) of the wall of the sample container; the second conduit connection (55) is passing through a second section (31b) of said wall; and at least a part of the three-way valve (5) is positioned in the interior (33) of the sample container (3) between said first and second section of the wall of the sample container.

## Description

The present invention relates to sampling device, comprising: a sample container having a wall inclosing an interior of the sample container, at least a part of said wall being flexible; and a three-way valve provided with a first conduit connection to a first coupling member; a second conduit connection to an outlet; and a connection to the interior of the sample container, whereby the three-way valve is configured to open and close at least a connection between the first coupling member and the interior of the sample container. The interior of the sample container is for containing a sample in use of the device. The first coupling member is configured to provide for connection to a sample outlet, either directly or through connecting means.

In general, the present invention relates to a sampling device for sampling e.g. through a sampling valve attached to a contents container used in an industrial processing plant, and preferably through a sample outlet connector attached to said sampling valve downstream of said sampling valve, a sample representative of a content of said contents container.

A sampling device of the above art is e.g. known from WO 2011/029450 A1, incorporated herein in its entirety by reference, which discloses a sampling device comprising a flexible bag with a tube connecting the bag and a connector for connection with a sample outlet, and a three-way valve inserted in the tube between the connector and the bag.

The tube of the known sampling device constitutes a dead volume of the overall sampling device and entails the use of an amount of material. Since the sampling device is most often used as a disposable device being used only once it would be beneficial and it is a general goal to be able to reduce the amount of material used for the sampling device and also the reduction of dead volumes would be beneficial and is a general goal.

It is an object of the present invention to provide a sampling device that fulfils at least some of the above goals.

This is obtained by a sampling device of the kind mentioned by way of introduction, wherein the first conduit connection to the first coupling member is passing through a first section of the wall of the sample container; the second conduit connection to the outlet is passing through a second section of the wall of the sample container; and at least a part of the three-way valve is positioned in the interior of the sample container between said first and second section of the wall of the sample container. Hereby is obtained that at least the piece of tube between the three-way valve and the sample container is avoided, in comparison with the above known sampling device, thus reducing the use om material for and the dead volume of the sampling device.

In an embodiment the three-way valve comprises two valve parts attachable to and detachable from each other, a first valve part of the two valve parts being attached to the first section of the wall and a second valve part of the two valve parts being attached to the second section of the wall, whereby the connection to the interior of the sample container is open when the two valve parts are detached from each other, and the connection to the interior is closed when the two valve parts are attached to each other. Hereby an unrestricted opening from the first conduit connection to the interior of the sample container may be obtained.

In a practical embodiment one of the two valve parts comprises a male part, the first or the second conduit connection extending through said male part, and the other of the two valve parts comprises a female part, the second or the first conduit connection extending through said female part, whereby, when the two valve parts are attached to each other, the male part is inserted in the female part and the first and second conduit connections are connected to provide an assembled conduit connection from the first coupling member to the outlet. Hereby an efficient sealing of the interior of the sample container is obtained when the two valve parts are attached to each other.

In an embodiment the sampling device comprises a locking device for securing the two valve parts in positions attached to each other. Hereby is obtained that the two valve parts do not unintended separate and open the connection to the interior of the sample container.

In an embodiment the locking device comprises an external clamp.

In another embodiment the locking device provides for mutual engagement by mutual rotation, i.e. rotary engagement, of the two valve parts. Hereby a compact device more simple to construct is obtained.

In a further embodiment the locking device is provided by one of the two valve parts comprising a first part of a bayonet mount or a thread and the other of the two valve parts is comprises a corresponding second part of a bayonet mount or a thread. Hereby an effective, compact locking device is obtained.

In yet a further embodiment at least one of the two valve parts comprises a rotatable portion provided for rotary engagement with the other of the two valve parts. Hereby handling of the device is facilitated.

In an embodiment the outlet is provided by a second coupling member. Hereby it is possible to connect a container for collecting a spend cleaning fluid during use of the sampling device.

In an embodiment at least one of the first coupling member and the outlet is provided with a removable sealing element sealing the respective first or second conduit connection. Hereby is provided for maintaining for the sampling device hygienic until use, and the sealing elements may function as tamper-evident features. Such sealing elements may e.g. be constituted by peel-off film or foil pieces sealing respective external openings of the first coupling member and the outlet.

In an embodiment a septum is provided in the wall of the sample container. Hereby a possibility of sub-sampling from the sample container is provided.

In the following the invention will be explained in further detail by means of non-limiting examples of embodiments having reference to the accompanying schematic drawings, in which
Fig. 1 shows a first embodiment of a sampling device according to the invention;
Fig. 2 is a side view of the valve of the sampling device of Fig. 1 and additional couplings;
Fig. 3 is an enlarged sectional view of the valve of Fig. 2 in a closed position;
Fig. 4 is a view corresponding to the view of Fig. 3 but showing the valve in an open position;
Fig. 5 is a perspective view of a second embodiment of a sampling device according to the invention;
Fig. 6 is an exploded view of the sampling device of Fig. 5; and
Fig. 7 is a sectional side view of the valve of the sampling device of Fig. 5.

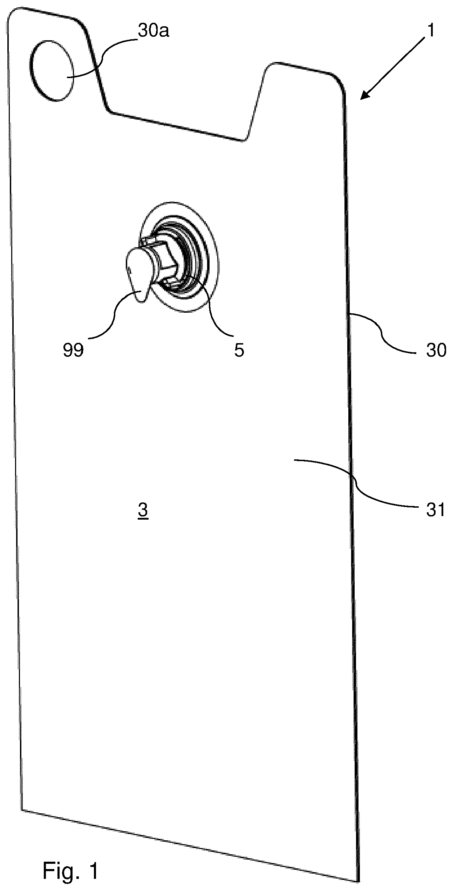

Fig. 1 shows a sampling device 1 comprising a sample container 3 and a three-way valve 5.

In the present embodiment, the sample container 3 comprises a bag of a suitable plastics material, possible a laminate, known in the art, the bag thus providing a flexible wall 31 of the sample container 3. In the present embodiment, the bag comprises two sheets of said plastics material said sheets being welded together along an edge 30 of the bag. A through hole 30a is provided for suspending the bag on a peg or the like. The two sheets are welded together at the edge of the hole. Thus the hole does not provide an opening from the interior of the sample container to the exterior.

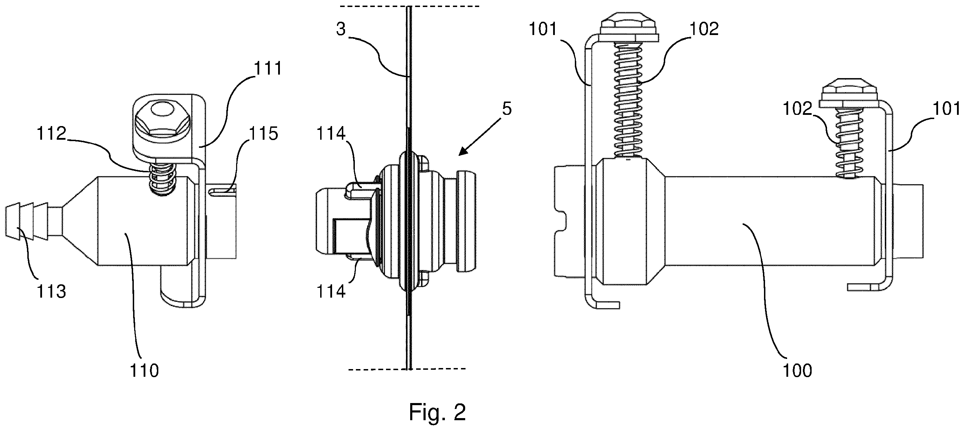

The three-way valve 5 is seen from an outlet side in Fig. 1 and the three-way valve is shown in more detail in Figs. 2-4.

Thus, the three-way valve 5 comprises a first valve part 7 with a first conduit connection 51 providing for fluid connection between a first coupling member 53 and an interior 5a of the three-way valve 5, and a second valve part 9 comprising a second conduit connection 55 providing for fluid connection between the interior 5a of the three-way valve 5 and a second coupling member 56 providing an outlet 57 of the three-way valve 5.

The first and the second valve part 7, 9, respectively, comprises a flange 7', 9' welded together with respective sheets of the bag to provide a first section 31a and a second section 31b of the wall of the sample container 3. The sample container 3 comprises an interior 33 for accommodating, in use, a sample (to be explained below) and part of said interior 33 is situated between the first section 31 a and the second section 31b of the wall of the sample container.

In the present embodiment, the second valve part 9 comprises a male part 91 with an external sealing ring 93 seated in a groove. The second conduit connection 55 passes through said male part 91. The first valve part 7 is provided to comprise a female part 71 comprising at least a section of the first conduit connection 51. The female part 71 is adapted to receive the male part 91 so that the sealing ring 93 seals any passage between the male part 91 and the female part 71.

In the present embodiment, the second valve part 9 comprises a first portion 95 comprising the flange 9' and a second portion 96 rotatably seated in the first portion 95 to rotate around an axis A. A sealing ring 97 seals between the first and the second portion 95, 96. The second portion 96 comprises the male part 91 and the second coupling member 55.

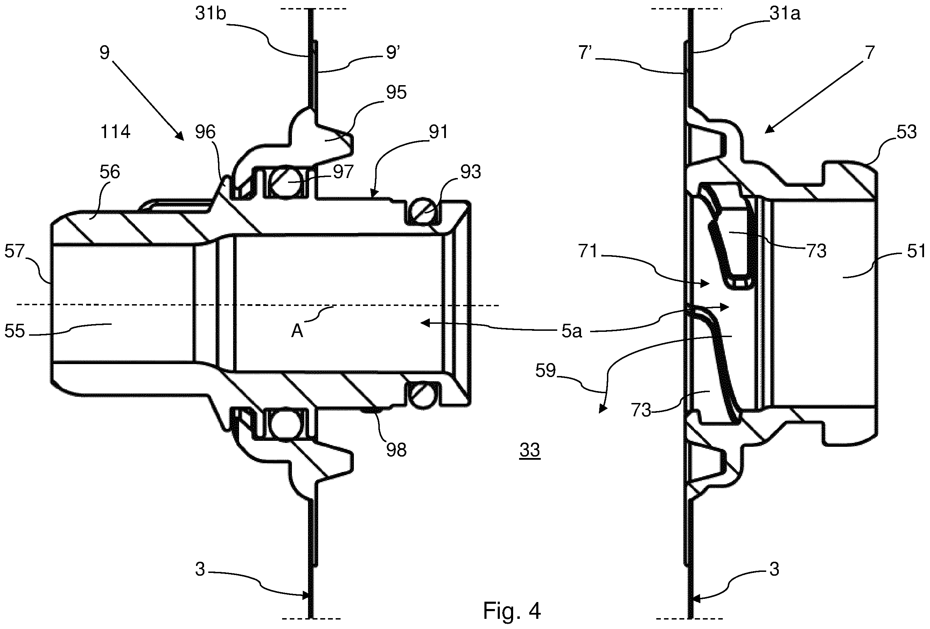

In the present embodiment, a locking device for securing the two valve parts 7, 9 in positions attached to each other is provided in that the female part 71 comprises grooves 73 constituting a first part of a bayonet mount and the male part 91 comprises lugs 98, of which only one is seen in Fig. 4, constituting a second part of a bayonet mount. Thus, it is possible to fasten the two valve parts 7, 9 to each other by means of the bayonet mount thus provided. As an alternative to the bayonet mount of the embodiment shown, the female part 71 might have an internal thread and the male part 91 might have an external thread for the male part and the female part to be threaded together to fasten the two valve parts 7, 9 to each other.

It is noted that instead of the second valve part 9 having a rotatable portion, the first valve part 7 might be provided with a rotatable portion to enable the bayonet mount or a threaded connection to be activated.

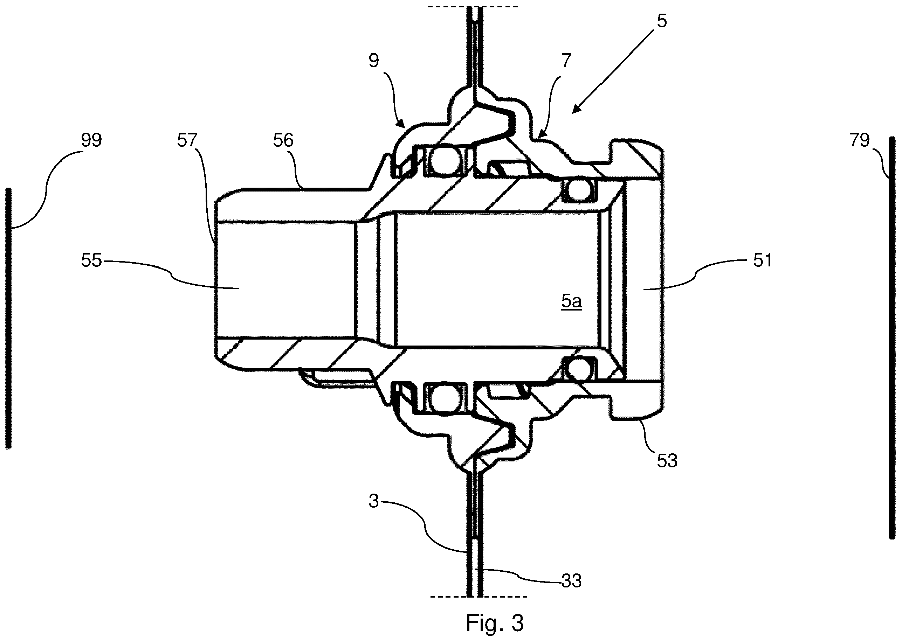

In Fig. 1 a piece of peel-off foil 99 is sealing the outlet 57. In Fig. 3 the piece of peel-off foil 99 is shown to be detached form the outlet and another piece of peel-off foil 79 is shown to be detached form the end of the first coupling member 53. It should be understood that the two pieces of peel-off foil 79 and 99, when attached to the outlet 57 and the first coupling member 53, respectively, close and seal the first conduit connection 51 and the second conduit connection 55 whereby sanitary conditions for the three-way valve 5 and the sample container 3 is assured and the two pieces of peel-off foil 79, 99 function as tamper evident features showing, when un-detached, that the sampling device has not been used.

In Fig. 2 the three-way valve 6 is shown together with external couplings of kinds known per se in the art.

Thus, a first coupling 100 is attachable on one hand to the first coupling member 53 and on the other hand to a sample outlet, not shown, from where a sample is to be obtained. The first coupling 100 comprises slides 101 that may be pushed manually against the action of springs 102 to allow the first coupling 100 to be applied to the first coupling member 53 and the sample outlet, respectively, whereby the first coupling 100 will be locked to the respective first coupling member 53 and the sample outlet on release of the respective slide 101 for said slide to return to the position shown in Fig. 2.

A second coupling 110 is attachable to the second coupling member 56 and likewise comprises a slide 111 and a spring 112 for locking the second coupling 110 to the second coupling member 56 and releasing it therefrom. The second coupling 110 further comprises a hose connector 113 for connecting a hose, not shown, to the outlet 57.

The rotatable second portion 96 of the second valve part 9 is provided with two lobes 114 that may be accommodated in corresponding slits 115 in the second coupling 110, when the latter is attached to the second coupling member 56, to rotationally lock the second coupling 110 to the rotatable second portion 96. Thus the second coupling 110 may be used as a handle for rotating the second portion 96 and as a visual indicator of the rotational position of the second portion. Thus, the second coupling 110 may provide a clear visual indication about the position of the bayonet mount and whether the first valve part 7 and the second valve part 9 are locked together.

It should be noted that in a starting position shown in Figs. 2 and 3, and possibly with peel-off foils 79, 99 attached to the first coupling member 53 and the outlet 57, respectively, the sampling device is provided in a condition in which the interior 33 of the sample container 3 is in a predetermined condition, e.g. empty and clean, i.e. sterile; hygienic; and/or sanitary to ensure that a sample to be received in the interior of the sample container is not contaminated by the sampling device. Likewise, the three-way valve 5 is empty and clean between the two peel-off foils 79, 99, if provided, prior to their removal or detachment.

The sampling device 1 may be used as follows:
Referring to the above WO 2011/029450 A1, incorporated herein by reference, with the three-way valve 5 in the position shown in Figs. 2 and 3, the two valve parts 7 and 9 being interconnected and locked to each other, the first coupling member 53 may be connected to a sample outlet connector at a sample outlet, not shown, by means of the first connector 100. Preferably the outlet 57 is connected to the second connector 110. These connections are performed after removal of any pieces of peel-off foils 79, 99. Preferably, a hose connected to a waste collection bag is attached to the hose connection 113.

Thus connected, a cleaning fluid, such as steam, may be flowed from the sample outlet connector, through the first connector 100; through the three-way valve 5, i.e. through the first conduit connection 51 and the second conduit connection 55; the second connector 110 and into the waste collection bag, if provided. Hereby a flow way from the sample outlet to the outlet 57 of the three-way valve 5 is cleaned and/or sterilized. The flow of cleaning fluid is stopped, and subsequently the two valve parts 7 and 9 are taken apart, possibly after an initial amount of a fluid to be sampled has been flowed through the connection from the sample outlet to the three-way valve 5, whereby a connection 59 from the interior 5a of the three-way valve 5, and thus from the sample outlet, to the interior 33 of the sample container is opened and a sample is introduced into the interior 33 of the sample container.

When a sufficient amount of fluid has been sampled, the sample outlet is shut, the three-way valve is closed by fitting together, and fastening to each other, the first valve part 7 and the second valve part 9, whereby the sample now accommodated in the interior 33 of the sample container is isolated from the exterior. If wanted, cleaning fluid may again be flowed through the connection from the sample outlet connector to the outlet 57 and possibly into the waste collection bag to avoid sample fluid dripping e.g. from the outside of the sample device when subsequently the sample device is detached from the first and second connector 100 and 110.

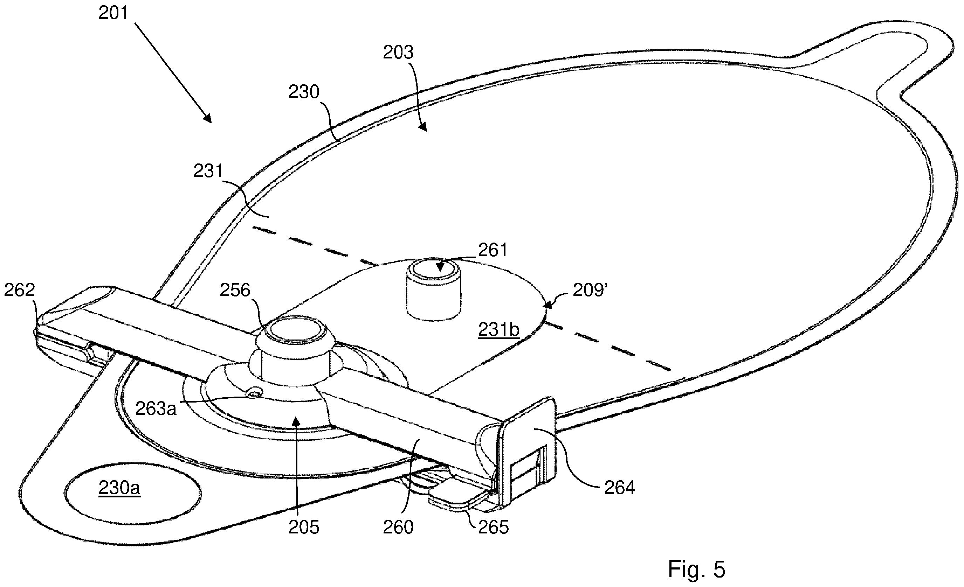

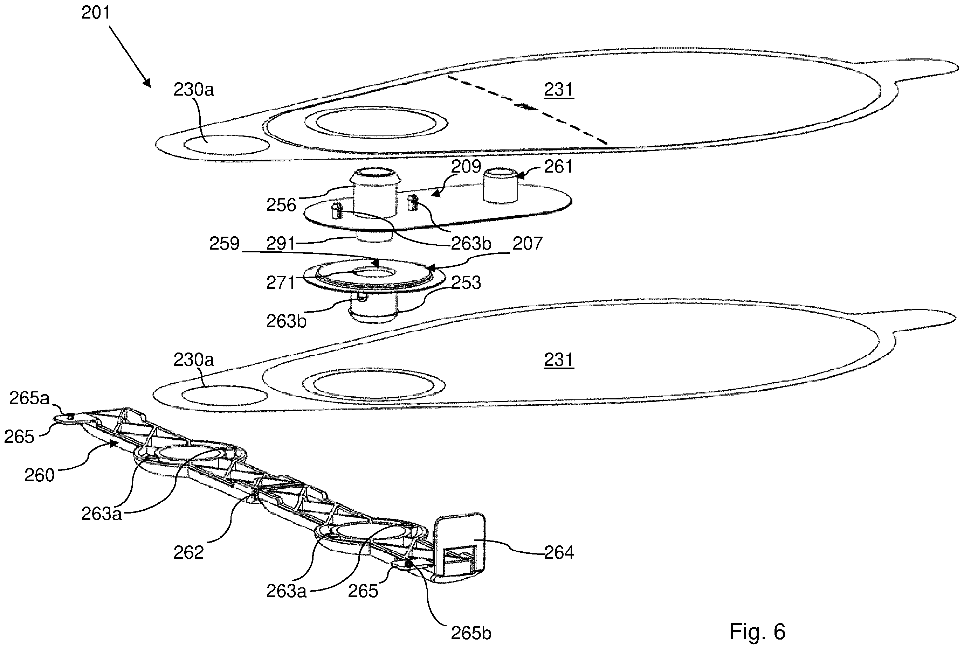

Figs. 5 to 7 show a second embodiment of a sampling device 201 according to the present invention. Similar elements are designated by similar numbers with the addition of 200 compared to elements of the first embodiment.

Thus, the sampling device 201 comprises a sample container 203 provided by two pieces of flexible sheet material, such as suitable plastics material, possible of laminate, known in the art, thus providing a flexible wall 231 of the sampling container 203. The two pieces of flexible sheet material are welded together by a seam 230 along the edge of the sample container 203. Outside the seam 230 a through hole 230a is provided for suspending the sampling device 201 e.g. on a peg (not shown).

Further the sampling device 201 comprises a three-way valve 205 which per se comprises a first valve part 207 and a second valve part 209.

The first valve part 207 comprises a flange 207' for welding together with the flexible material to form a first section 231a of the wall of the sample container 203 and the second valve part 209 comprises another flange 209' for welding together with the flexible material to form a second section 231b of the wall of the sample container 203.

The three-way valve 205 is provided with a first conduit connection 251 to a first coupling member 253; a second conduit connection 255 to an outlet 257; and a connection 259 to an interior 233 of the sample container. The three-way valve 205 is configured to open and close a connection between the first coupling member 253 and the interior 233 of the sample container 203.

Like in the first embodiment, the first conduit connection 251 to the first coupling member 253, in the present embodiment a hose connector, is passing through the first section 231a of the wall of the sample container; the second conduit connection 255 to the outlet 257 is passing through the second section 231b of the wall of the sample container; and a part of the three-way valve 205 is positioned in the interior 233 of the sample container 203 between said first and second section of the wall of the sample container.

The two valve parts 207, 209 are attachable to and detachable from each other, whereby the connection 259 to the interior 233 of the sample container is open when the two valve parts 207, 209 are detached from each other, and the connection 259 to the interior is closed when the two valve parts 207, 209 are attached to each other.

Further. like in the first embodiment one of the two valve parts 207, 209, in the present embodiment the second valve part 209, comprises a male part 291 for the second conduit connection 255 to extend through said male part 291, and the other of the two valve parts 207, 209, i.e. the first valve part 207 in the present embodiment, comprises a female part 271 for the first conduit connection 251 to extend through said female part 271, whereby, when the two valve parts 207, 209 are attached to each other, the male part 291 is inserted in the female part 271 and the first and second conduit connections 251, 255 are connected to provide a conduit connection from the first coupling member 253 to the outlet 257 and to close the connection 259 between the first coupling member 253 and the interior 233 of the sample container.

In order to ensure that the connection 259 from an interior 205a of the three-way valve 205 to the interior 233 of the sample container is effectively closed when the first and the second valve parts 207. 209 are attached to each other, the male part 291 and the female part 271 are in the present embodiment shaped with a taper corresponding to a Luer taper.

In the present embodiment the sampling device 201 comprising a locking device in the form of an external clamp 260 for securing the two valve parts 207, 209 in positions attached to each other.

In the present embodiment, the outlet 257 is provided by a second coupling member in the form of a second hose connector 256.

In the present embodiment, the flange 209' of the second valve part 209 incorporates a septum 261 to provide for insertion of a syringe needle to take a sample of a sample provided in the sample container 203.

The external clamp 260 is provided with two halves interconnected by a hinge part 262. The two halves are provided with holes 263a for receiving barbed pegs 263b provided on the flanges 207', 209', whereby the two halves are fastened respectively to the flanges 207', 209' when the external clamp 260 is folded into the position shown in Fig. 5.

The external clamp 206 further comprises a releasable catch 264 and taps 265 of which one is provided with a barbed peg 265a and the other is provided with a hole 265b for receiving the barbed peg 265a in a non-release manner. Accordingly, when the sampling device 201 is assembled to the configuration shown in Fig. 5, the external claim 206 is locked in the closed position shown and likewise the two valve parts 207, 209 are attached to each other the connection 259 to the interior 233 of the sample container 203 being closed.

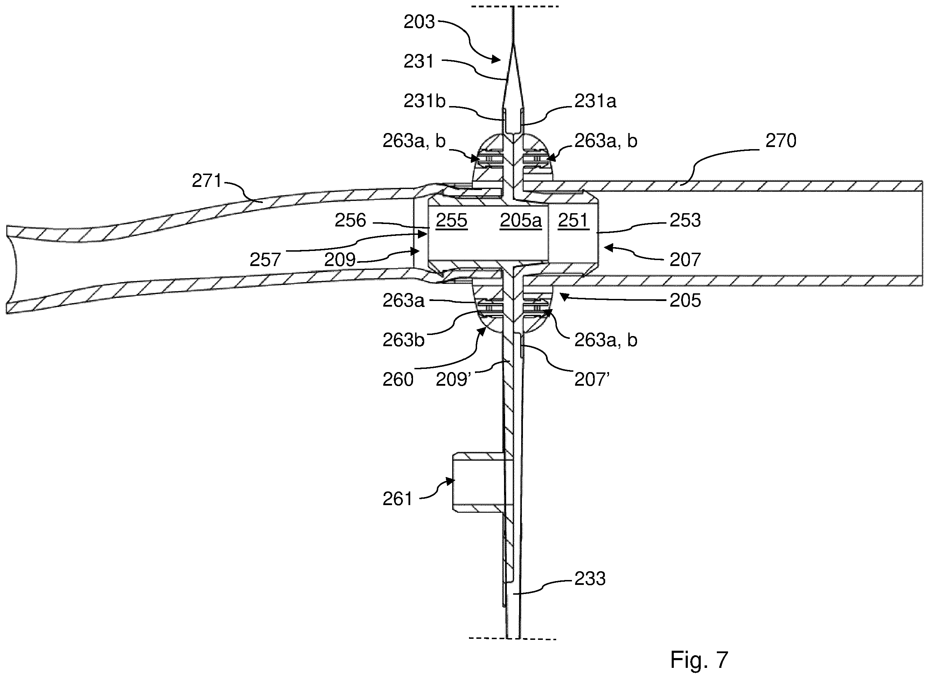

In order to use the sampling device 201, basically in a manner similar to the manner of use of the first embodiment including connecting the sampling device 201 to a sample outlet (not shown), in the present embodiment by means of a hose 270 (see Fig. 7) connected to the first hose connector 253, and flushing the interior 205a of the three-way valve 205 with e.g. steam, the tabs 265 need to be broken off prior to opening the external clamp 260 by releasing (i.e. pushing aside) the releasable catch 264 and pulling apart the two halves of the external clamp and therewith the two valve parts 207 and 209 in order to open the connection 259 from the first conduit connection 251 to the interior 233 of the sample container 203.As shown in Fig.7 a second hose 271 is connected to the second coupling member 256 and said second hose 271 may further be connected to a waste collection bag as with the first embodiment.

It should be noted that if it is desired to close the outlet 57, 257 this may be done by attaching an ordinary hose claim to the hose connected to the second coupling member 56, 256, e.g. via the second coupling 110 of the first embodiment. Such hose claim in known in the art to provide for squeezing flat a hose to which it is applied and prevent flow through the hose.

## Claims

1. A sampling device (1; 201), comprising:
a sample container (3; 201) having a wall inclosing an interior (33; 233) of the sample container, at least a part (31; 231) of said wall being flexible; and
a three-way valve (5; 205) provided with a first conduit connection (51; 251) to a first coupling member (53; 253); a second conduit connection (55; 255) to an outlet (57; 257); and a connection (59; 259) to the interior (33; 233) of the sample container, whereby the three-way valve (5; 205) is configured to open and close at least a connection between the first coupling member (53; 253) and the interior (33; 233) of the sample container (3; 203), **characterized in that** the first conduit connection (51; 251) to the first coupling member (53; 253) is passing through a first section (31a; 231a) of the wall of the sample container; the second conduit connection (55; 255) to the outlet (57; 257) is passing through a second section (31b; 231b) of the wall of the sample container; and at least a part of the three-way valve (5; 205) is positioned in the interior (33; 233) of the sample container (3; 203) between said first and second section of the wall of the sample container.

2. A sampling device according to claim 1, wherein the three-way valve comprises two valve parts (7, 9; 207, 209) attachable to and detachable from each other, a first valve part (7; 207) of the two valve parts being attached to the first section (31a; 231a) of the wall and a second valve part (9; 209) of the two valve parts being attached to the second section (31b; 231b) of the wall, whereby the connection (59; 259) to the interior (33; 233) of the sample container is open when the two valve parts (7, 9; 207, 209) are detached from each other, and the connection (59; 259) to the interior is closed when the two valve parts (7, 9; 207, 209) are attached to each other.

3. A sampling device according to claim 2, wherein one of the two valve parts (7, 9; 207, 209) comprises a male part (91; 291) for the first or the second conduit connection to extend through said male part, and the other of the two valve parts (7, 9; 207, 209) comprises a female part (71; 271) for the second or the first conduit connection to extend through said female part, whereby, when the two valve parts (7, 9; 207, 209) are attached to each other, the male part is inserted in the female part and the first and second conduit connections are connected to provide a conduit connection from the first coupling member (53; 253) to the outlet (57; 257) and to close the connection between the first coupling member (53; 253) and the interior (33; 233) of the sample container.

4. A sampling device according to claim 2 or 3, comprising a locking device for securing the two valve parts (7, 9; 207, 209) in positions attached to each other.

5. A sampling device according to claim 4, wherein the locking device comprises an external clamp (260).

6. A sampling device according to claim 4, wherein the locking device provides for mutual engagement by mutual rotation of the two valve parts (7, 9).

7. A sampling device according to claim 6, wherein the locking device is provided by one of the two valve parts (7, 9) comprising a first part (73) of a bayonet mount or a thread and the other of the two valve parts is comprises a corresponding second part (98) of a bayonet mount or a thread.

8. A sampling device according to claim 6 or 7, wherein at least one of the two valve parts (7, 9) comprises a rotatable portion (96) provided for rotary engagement with the other of the two valve parts.

9. A sampling device according to any one of claims 1 to 8, wherein the outlet (57; 257) is provided by a second coupling member (56; 256).

10. A sampling device according to any one of claims 1 to 9, wherein at least one of the first coupling member and the outlet is provided with a detachable sealing element, such as a peel-off foil (79, 99), sealing the respective first or second conduit connection.

11. A sampling device according to any one of claims 1 to 10, wherein a septum (261) is provided in the wall of the sample container.
